# EUROPÄISCHE PATENTSCHRIFT

(11) **EP 1 300 114 B1**
(45) Veröffentlichungstag und Bekanntmachung des Hinweises auf die Patenterteilung: **17.11.2004**
(21) Anmeldenummer: 02021517.4
(22) Anmeldetag: 26.09.2002
(51) Int. Cl.: A61B 5/15

(54) **Evakuierungseinrichtung für ein Blutentnahmeröhrchen**
Evacuating device for a blood collection tube
Dispositif pour évacuer des tubes de prélèvement de sang

(30) Priorität: 06.10.2001 DE 10149435
(43) Veröffentlichungstag der Anmeldung: 09.04.2003
(73) Patentinhaber: Sarstedt AG & Co., 51588 Nümbrecht (DE)
(72) Erfinder: Sarstedt, Walter, 51588 Nümbrecht (DE)
(74) Vertreter: Valentin, Ekkehard, Dipl.-Ing.

(56) Entgegenhaltungen:
- WO-A-88/07351
- US-A- 4 327 746
- US-A- 4 699 190
- US-A- 5 313 969

## Beschreibung

Die Erfindung betrifft eine Evakuierungseinrichtung zur Erzeugung eines Vakuums in einem Blutentnahmeröhrchen, umfassend in einem Gerätegehäuse eine Vakuumpumpe mit einer daran über eine Leitung angeschlossenen Saugnadel, die beim Aufstecken des Blutentnahmeröhrchens in eine Einführaufnahme die Röhrchenmembrane durchstößt, und eine mit der Vakuumpumpe schaltungstechnisch verbundene Druckmeßeinrichtung.

Eine solche Evakuierungseinrichtung für ein Blutentnahmesystem ist durch die DE 29 08 817 A1 bekanntgeworden und dazu geeignet, erst kurz vor bzw. zum Zeitpunkt der Anwendung, z.B. am Anfang eines Tages für einen Tagesbedarf, ein Vakuum in den benötigten Blutentnahmeröhrchen zu erzeugen. Die Evakuierungseinrichtung besitzt in einem Gerätegehäuse eine hohle Saugnadel, die über eine ein Druckmeßgerät aufweisenden Leitung mit der durch einen Elektromotor angetriebenen Vakuumpumpe verbunden ist. Die Saugnadel wird von einem Einführungstrichter umschlossen, in den das Blutentnahmeröhrchen hieneingeschoben wird, bis die Nadelspitze die das Röhrcheninnere abdichtende Membran durchstößt. Bei einer bestimmten Anschlaglage des Blutentnahmeröhrchens in dem Einführungstrichter wird die Vakuumpumpe eingeschaltet und drucküberwacht erst dann abgeschaltet, wenn das in der Leitung überwachte Vakuum eine bestimmten Soll-Wert erreicht hat. Sobald das dann einsatzbereite Blutentnahmeröhrchen von der Saugnadel abgezogen wird, ist die Evakuierungseinrichtung für einen nächsten Arbeitsgang bereit. Aus Dokument US 5 313 969 ist darüber hinaus bekannt den Einführungstrichter nach Beendigung des Evakuierungsvorganges durch eine Kraft in die Ausgangslage Zurückzubringen. Diese Geräte besitzen aber Nachteile bei der Handhabung zur Erzeugung des Vakuums in Blutentnahmeröhrchen, insbesondere dann, wenn diese mit einer Präparierung befüllt sind und/oder wenn unterschiedlich lange Blutentnahmeröhrchen evakuiert werden sollen.

Der Erfindung liegt die Aufgabe zugrunde, eine gattungsgemäße Evakuierungseinrichtung zu schaffen, die das Evakuieren betriebssicherer macht, insbesondere auch dann, wenn mit einer Präparierung befüllte und/oder unterschiedlich lange Blutentnahmeröhrchen verwendet werden.

Diese Aufgabe wird erfindungsgemäß dadurch gelöst, daß die Einführaufnahme mit einer von unten nach oben ansteigenden Schräglage im Gerätegehäuse verschiebbar ausgebildet ist und die Einführaufnahme nach Beendigung des Evakuierungsvorganges während oder nach dem Herausnehmen der Röhre durch eine Kraft, vorzugsweise durch eine Feder, in die Ausgangslage zurückgebracht wird. Die Einführaufnahme bietet trotz der Schräglage einen zur leichten Zugänglichkeit hinreichend großen Freiraum, und zwar auch dann, wenn die Evakuierungseinrichtung nicht wie bevorzugt an einer mit ihrem Einführtrichter über die Vorderkante einer Arbeitsplatte vorspringenden Lage positioniert wird. Die erfindungsgemäße Schräglage stellt dabei auf jeden Fall sicher, daß keine Präparierung aus dem zu evakuierenen Blutentnahmeröhrchen abgesaugt wird. Durch die definierte Verschiebbarkeit der Einführaufnahme gegen eine anstehende Kraft, wie eine Feder, ein Gegengewicht, ein Druckpolster oder dergleichen, wird erreicht, daß diese einerseits stets selbsttätig in die Ausgangsposition zurückgestellt wird und andererseits in der Saugposition eine ausreichende Grifflänge des Blutentnahmeröhrchens zur Verfügung steht, und zwar unabhängig von der jeweiligen Röhrchenlänge.

Weitere Merkmale und Einzelheiten der Erfindung ergeben sich aus dem Anspruch und der nachfolgenden Beschreibung von in den Zeichnungen schematisch dargestellten Ausführungsbeispielen der Erfindung. Es zeigen:
- Fig. 1: eine teilgeschnittene Seitenansicht einer ersten Ausführung einer Evakuierungseinrichtung, in der Ausgangsposition dargestellt;
- Fig. 2: die Evakuierungseinrichtung nach Fig. 1 in der Saugposition;
- Fig. 3: eine teilgeschnittene Seitenansicht einer anderen Ausführung einer Evakuierungseinrichtung, in der Ausgangsposition dargestellt; und
- Fig. 4: die Evakuierungseinrichtung nach Fig. 3 in der Saugposition.

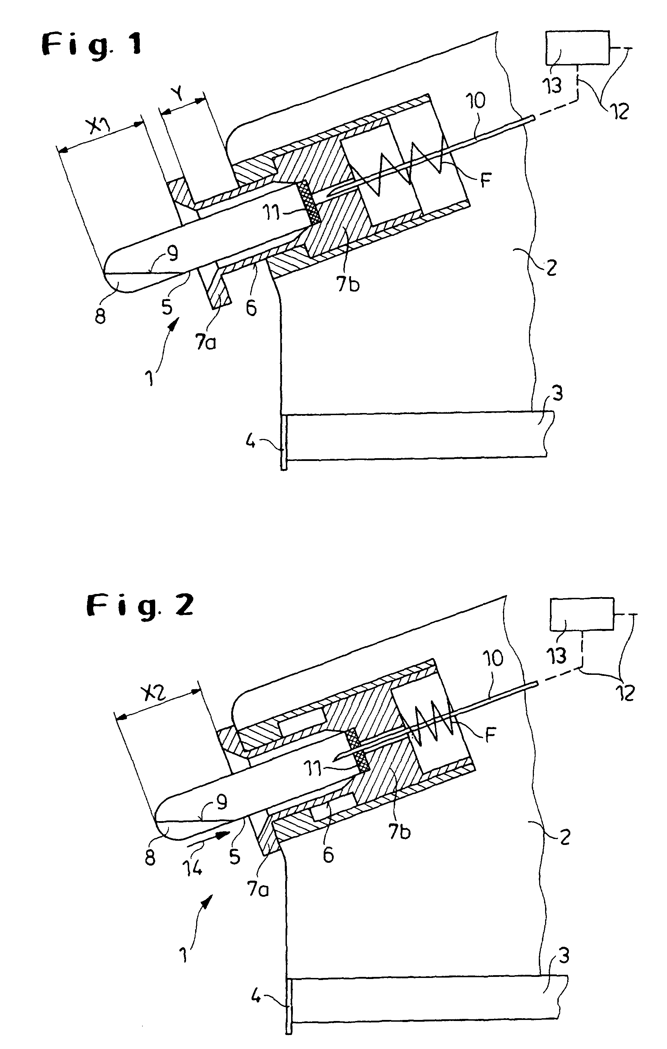

Eine in den Figuren 1 und 2 dargestellte Evakuierungseinrichtung 1 ist mit ihrem Gerätegehäuse 2 auf einer Arbeitsplatte 3 angeordnet. Hierbei gewährleistet eine sich an die Stirnfläche der Arbeitsplatte 3 anlegende Anschlagkante 4 des Gerätegehäuses 2 eine genaue Positionierung. Ein Festschrauben und/oder Klemmen der Evakuierungseinrichtung 1 ist damit entbehrlich. Alternativ und/oder ergänzend könnte die Evakuierungseinrichtung 1 auch auf einer rutschfesten Unterlage der Arbeitsplatte 3 aufgestellt werden.

Eine Einführaufnahme 6 zur Aufnahme eines zu evakuierenden Blutentnahmeröhrchens 5 besteht aus einem Einführtrichter 7a, der in diesem Ausführungsbeipiel einstückig mit einer Zentrieraufnahme 7b ausgebildet ist. Sie ist in dem Gerätegehäuse 2 mit einer von unten nach oben, d.h. vom äußeren zum inneren Ende ansteigenden Schräglage angeordnet. Diese Neigung ermöglicht es, daß eine Präparierung 8 weitestgehend im Endbereich am Boden des Blutentnahmeröhrchens 5 verbleibt, wie durch den Preparierungsspiegel 9 verdeutlicht, und daher nicht in den Wirkbereich einer zur Erzeugung des Vakuums in das Röhrcheninnere hineinragenden Saugnadel 10 gelangen kann; ein An- bzw. Absaugen von Präparierung wird somit verhindert.

Die - wie in Figur 2 dargestellt - beim Einführen des Blutentnahmeröhrchens 5 in den Einführtrichter 7a und die Zentrieraufnahme 7b in der Saugposition eine Röhrchenmembrane 11 durchstoßende Saugnadel 10, wobei der Einführtrichter 7a und die Zentrieraufnahme 7b einen Weg Y zurücklegen, ist über eine Anschlußleitung 12 mit einer von einem Elektromotor (nicht gezeigt) angetriebenen Vakuumpumpe 13 verbunden, die drucküberwacht dann abgeschaltet wird, wenn in dem Blutentnahmeröhrchen 5 das gewünschte Vakuum vorliegt.

Die in der Schräglage angeordnete Einführaufnahme 6 wird von einer Druckkraft F, z.B. eine Druckfeder, in der Ausgangsposition nach Figur 2 in einer aus dem Gerätegehäuse 2 vorkragenden Position gehalten. Auch nach dem Einschieben des Blutentnahmeröhrchens 5 bzw. des Einführtrichters 7a samt Zentrieraufnahme 7b, wobei ab einem gewissen Punkt gegen einen Widerstand der Pumpenmechanismus aktiviert wird, in Pfeilrichtung 14 gegen die Druckkraft F in das Gerätegehäuse 2, steht für eine Bedienungsperson eine ausreichende Grifflänge zur Verfügung. Denn die Grifflänge X2 eines Blutentnahmeröhrchens 5 in der Saugposition (Fig. 2) entspricht der Grifflänge X1 dieses Blutentnahmeröhrchens 5 in der Ausgangsposition (vgl. Fig. 1).

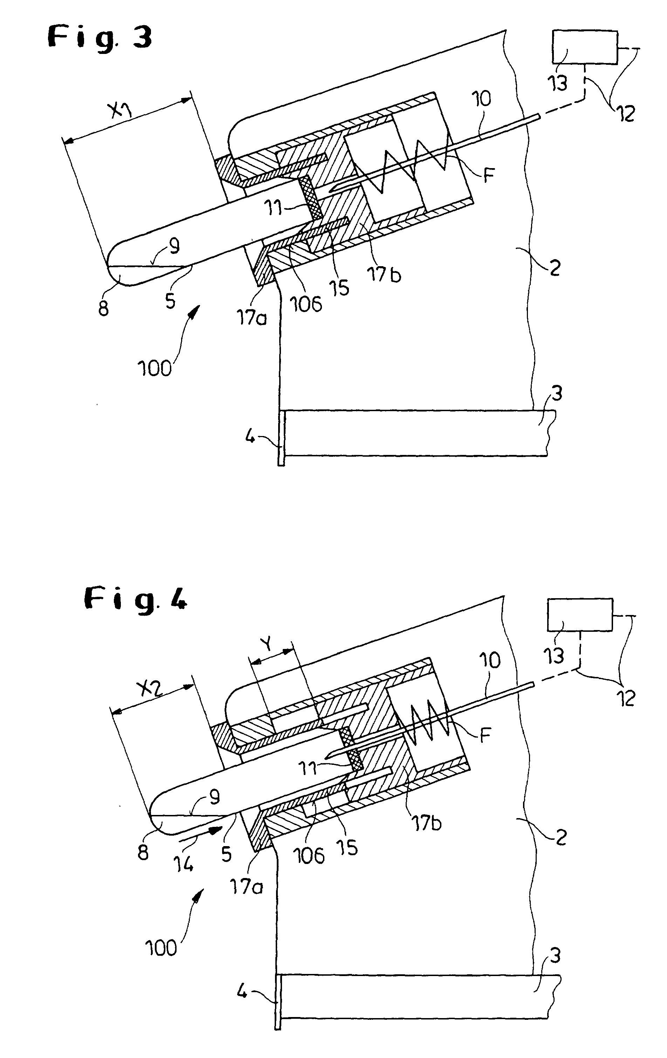

Bei einer anderen, in den Figuren 3 und 4 dargestellten Ausführung einer Evakuierungseinrichtung 100 liegt als Unterschied zu der vorbeschriebenen Ausführung, so daß gleiche Bauteile mit denselben Bezugsziffern versehen sind, lediglich vor, daß der Einführtrichter 17a und die Zentrieraufnahme 17b der Einführaufnahme 106 zwei Bauteile sind, wobei in diesem Ausführungsbeispiel der Einführtrichter 17a ohne Bewegungmöglichkeit fest im Gerätegehäuse 2 angeordnet und der Verschiebeweg Y in das Geräteinnere verlegt ist. Denn abweichend von der anderen Geräteausführung wird hier nur die auf einem gleichzeitig als Anschlag dienenden Stutzen 15 des Einführtrichters 17a geführte, den Trichterboden der Einführaufnahme 106 bildende Zentrieraufnahme 17b nach dem Einstecken des Blutentnahmeröhrchens 5 (vgl. die Ausgangsposition Fig. 3) gegen die Kraft F verschoben, wie der Saugposition mit aktiviertem Pumpenmechanismus nach Figur 4 zu entnehmen ist.

In allen Fällen besteht für eine Bedienungsperson keinerlei Verletzungsgefahr, da z.B. ein Finger nicht bis zum Bereich der Saugnadel 10 bzw. des Verschiebemechanismus gelangen kann und erst ein eingeschobenes Blutentnahmeröhrchen 5 die Saugnadel 10 freilegt, deren Spitze dann allerdings sogleich in das einen Schutz bietende Röhrcheninnere eindringt. Weiterhin ist die aus der Einführaufnahme 6 bzw. 106 herausragende freie Länge X1 und X2 des Blutentnahmeröhrchens 5 stets so ausreichend bemessen und durch die Anschläge zur Begrenzung der Verschiebebewegungen der Einführaufnahme 6 bzw.106 festgelegt, daß das Blutentnahmeröhrchen 5 injeder Position unverändert gut gefaßt werden kann.

## Patentansprüche

1. Evakuierungseinrichtung (1; 100) zur Erzeugung eines Vakuums in einem Blutentnahmeröhrchen (5), umfassend in einem Gerätegehäuse (2) eine Vakuumpumpe (13) mit einer daran über eine Leitung (12) angeschlossenen Saugnadel (10), die beim Aufstecken des Blutentnahmeröhrchens (5) in eine Einführaufnahme (6;17b) eine Röhrchenmembrane (11) durchstößt, eine mit der Vakuumpumpe (13) schaltungstechnisch verbundene Druckmeßeinrichtung,
wobei die Einführaufnahme (6; 17b) im Gerätegehäuse (2) verschiebbar ausgebildet ist und nach Beendigung des Evakuierungsvorganges während oder nach dem Herausnehmen des Blutentnahmeröhrchens (5) durch eine Kraft, vorzugsweise durch eine Feder, in die Ausgangslage zurückgebracht wird
**dadurch gekennzeichnet daß** die Einführaufnahme (6, 17b) mit einer von unter nach oben ansteigenden Schräglage im Gerätegehäuse ausgebildet ist.

## Claims

1. An evacuation device (1; 100) for producing a vacuum in a blood sampling tube (5), comprising a vacuum pump (13) in an equipment casing (2) with a suction needle (10) connected thereto via a lead (12), which pierces a tube membrane (11) when the blood sampling tube (5) is pushed into an insertion receiver (6; 17b), a pressure measuring device connected by circuitry to the vacuum pump (13), wherein the insertion receiver (6; 17b) is constructed displaceably in the equipment casing (2) and after the end of the evacuation process during or after removal of the blood sampling tube (5) is brought back into the starting position by a force, preferably by a spring,
**characterised in that** the insertion receiver (6, 17b) is constructed with a sloping position ascending from bottom to top in the equipment casing.

## Revendications

1. Dispositif de mise sous vide (1 ; 100) servant à générer un vide dans une pipette de prélèvement sanguin (5), comprenant dans un boîtier d'appareil (2) une pompe à vide (13) avec une aiguille d'aspiration (10) qui lui est raccordée par une conduite (12), lequel dispositif, lors de l'insertion de la pipette de prélèvement sanguin (5) dans un support d'introduction (6 ; 17b), transperce une membrane de la pipette (11) et un dispositif de mesure de pression relié par technique de circuits à la pompe à vide (13),
le support d'introduction (6 ; 17b) étant conçu déplaçable dans le boîtier de l'appareil (2) et étant ramené, après la fin de l'opération de mise sous vide, pendant ou après le retrait de la pipette de prélèvement sanguin (5), par une force, de préférence par un ressort, dans sa position initiale,
**caractérisé en ce que** le support d'introduction (6, 17b) est conçu avec une position oblique ascendante du bas vers le haut dans le boîtier d'appareil.
